(19) Europäisches Patentamt
European Patent Office
Office européen des brevets

(11) **EP 2 727 580 B1**

(12) **EUROPEAN PATENT SPECIFICATION**

(45) Date of publication and mention
of the grant of the patent:
**04.01.2017 Bulletin 2017/01**

(21) Application number: **12805020.0**

(22) Date of filing: **27.06.2012**

(51) Int Cl.:
*A61K 8/67* (2006.01)      *A61K 8/31* (2006.01)
*A61K 8/35* (2006.01)      *A61Q 19/00* (2006.01)
*A61Q 19/08* (2006.01)

(86) International application number:
**PCT/JP2012/066419**

(87) International publication number:
**WO 2013/002278 (03.01.2013 Gazette 2013/01)**

(54) **ASTAXANTHIN-CONTAINING COMPOSITION, METHOD FOR MANUFACTURING SAME, AND COSMETIC**

ASTAXANTHINHALTIGE ZUSAMMENSETZUNG, HERSTELLUNGSVERFAHREN DAFÜR UND KOSMETIKARTIKEL DAMIT

COMPOSITION CONTENANT DE L'ASTAXANTHINE, SON PROCÉDÉ DE FABRICATION, ET PRODUIT COSMÉTIQUE ASSOCIÉ

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **28.06.2011 JP 2011143444**

(43) Date of publication of application:
**07.05.2014 Bulletin 2014/19**

(73) Proprietor: **Fujifilm Corporation**
**Minato-ku**
**Tokyo 106-8620 (JP)**

(72) Inventors:
• **TATEISHI, Tomomi**
**Ashigarakami-gun**
**Kanagawa 258-8577 (JP)**

• **KUSUDA, Fumi**
**Ashigarakami-gun**
**Kanagawa 258-8577 (JP)**

(74) Representative: **Höhfeld, Jochen et al**
**Klunker Schmitt-Nilson Hirsch**
**Patentanwälte**
**Destouchesstrasse 68**
**80796 München (DE)**

(56) References cited:
WO-A1-2009/039716     WO-A1-2010/112071
JP-A- 10 324 816     JP-A- 2002 265 313
JP-A- 2007 160 287     JP-A- 2007 160 287
US-A1- 2010 267 838

• DATABASE GNPD [Online] MINTEL; 1 November 2004 (2004-11-01), "M.D. Formd Mist On Toner", XP002735049, Database accession no. 10194688

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

Technical Field

The invention relates to an astaxanthin-containing composition, a method for manufacturing the same, and a cosmetic preparation including the astaxanthin-containing composition.

Background Art

Focusing on high functions of carotenoids, the addition of carotenoids to foods, cosmetic products, pharmaceutical raw materials, processed goods thereof have been recently examined and carried out.

Astaxanthin which is a kind of carotenoid is widely distributed in animals and plants in nature, and is mainly used as a reviver for farmed fish or farm-raised chickens. Astaxanthin is known to have functions such as an antioxidant effect, an anti-inflammatory effect, an anti-skin aging effect, and an effect of preventing the formation of spots or wrinkles, and various compositions in which astaxanthin is applied are disclosed (see Japanese Patent Application Laid-Open (JP-A) Nos. H2-49091, H5-155736, 2005-47860, 2005-28559, and 2007-160287). Further, JP-A No. 2000-229827 discloses that a tomato pigment containing a lycopene complex exhibiting excellent solubility in an aqueous liquid as a main component has an effect of preventing the formation of lipid peroxide on the skin surface and in the skin, the skin inflammation, and aging phenomena such as tanning, wrinkles, and sagging.

SUMMARY OF INVENTION

Technical Problem

However, carotenoids such as astaxanthin and lycopene have an unstable structure and are easily oxidatively-decomposed. Therefore, a decrease in storage stability in a composition containing astaxanthin particularly raises a problem. Further, lycopene exhibits high crystallinity. In a case where a composition containing lycopene is left over time, crystals precipitat in the composition. This causes a problem of reducing the storage stability.

Consequently, improvement in storage stability of the compositions containing carotenoids such as astaxanthin and lycopene is desired. However, the improvement has not been achieved yet.

The invention has been made under such circumstances, and an object of the invention is to provide an astaxanthin-containing composition that is excellent in storage stability, a method for manufacturing the same, and a cosmetic preparation that is excellent in storage stability including the astaxanthin-containing composition.

Solution to Problem

The invention is as follows:

<1> An astaxanthin-containing composition including: 100 parts by mass of astaxanthin; 1 to 200 part by mass of lycopene; and 10 000 to 1000000 parts by mass of tocopherol where the content ratio of lycopene and tocopherol by mass is from 0.5 : 100000 to 10 : 200.
<2> The astaxanthin-containing composition according to <1>, wherein the lycopene is a fat-soluble extract derived from tomato pulp.
<3> The astaxanthin-containing composition according to <1> or <2>, wherein the astaxanthin-containing composition is an oil composition or emulsion composition.
<4> The astaxanthin-containing composition according to any one of <1> to <3>, further including an oil agent.
<9> A cosmetic preparation including the astaxanthin-containing composition according to any one of <1> to <4>.
<10> A method for manufacturing an astaxanthin-containing composition including: preparing an oil phase including 100 parts by mass of astaxanthin, 1 to 200 parts by mass of lycopene, and 10 000 to 1000000 parts by mass of tocopherol where the content ratio of lycopene and tocopherol by mass is from 0.5 to 100000 to 10 : 200 ; and mixing the prepared oil phase with an aqueous phase including an aqueous phase component to obtain an emulsion composition.

Advantageous Effects of Invention

According to the invention, an astaxanthin-containing composition that is excellent in storage stability, a method for manufacturing the same, and a cosmetic preparation that is excellent in storage stability including the astaxanthin-containing composition can be provided.

## DESCRIPTION OF EMBODIMENTS

The astaxanthin-containing composition of the invention is an astaxanthin-containing composition which includes (A) 100 parts by mass of astaxanthin, (B) 1 to 200 parts by mass of lycopene, and (C) 10 000 to 1000000 parts by mass of tocopherol.

The astaxanthin-containing composition of the invention contains specific amounts of astaxanthin, lycopene, and tocopherol, whereby the decomposition of astaxanthin and lycopene and the precipitation of the crystal of lycopene in the system can be effectively suppressed. As a result, the astaxanthin-containing composition of the invention has excellent storage stability.

The term "step" used herein includes not only a discrete step, but also a step which cannot be clearly distinguished from another step, as long as a desired effect of the step can be achieved.

Any numerical range expressed herein using "to" refers to a range including the numerical values before and after the "to", as a minimum value and a maximum value, respectively.

Furthermore, in a case in which the amount of a component in the composition is indicated in the specification, when there are plural substances corresponding to the component in the composition, the indicated amount means the total amount of the plural substances present in the composition, unless specifically stated otherwise.

The term "LogP value" used herein means the calculated octanol-water partition coefficient (cLogPow).

The term "HLB" used herein means a hydrophilic-lipophilic balance.

Hereinafter, the invention will be described.

[Astaxanthin-containing composition]

The astaxanthin-containing composition of the invention (hereinafter also appropriately referred to as a "composition of the invention") contains (A) 100 parts by mass of astaxanthin, (B) 1 to 200 parts by mass of lycopene, and (C) 10000 to 1000000 parts by mass of tocopherol.

The respective components - (A) astaxanthin, (B) lycopene and (C) tocopherol - are contained in the composition of the invention as oil components.

Examples of the form of the composition of the invention include a composition in oil form, a composition in emulsified form, and a composition in powder form. The composition in emulsified form is preferably an oil-in-water type emulsion composition. The composition in powder form can be prepared, for example, by drying the composition in emulsified form.

Hereinafter, essential components and optional components in the composition of the invention will be described.

(A) Astaxanthin

In the invention, the term "astaxanthin" encompasses at least one of astaxanthin or a derivative thereof such as an astaxanthin ester. Unless otherwise noted, these components are collectively referred to as "astaxanthin" herein.

Besides those derived from natural materials such as plants, algae, and bacteria, any as astaxanthin that can be obtained by an ordinary method can be used as the astaxanthin for use in the invention.

Examples of the natural materials include red yeast phaffia, green alga Haematococcus, marine bacteria, and krill. Further, examples thereof may include extracts from extracts or the like obtained from culture products thereof.

The astaxanthin may be included in the composition of the invention, as astaxanthin-containing oil obtained by separation or extraction from natural materials containing astaxanthin. The astaxanthin may be obtained by appropriately purifying a product separated or extracted from the natural material as needed or a synthetic product.

The astaxanthin is particularly preferably an extract from a Haematococcus algae (also referred to as a "Haematococcus algae extract") or a pigment derived from krill from the viewpoint of quality and productivity.

Specifically, examples of the astaxanthin derived from the Haematococcus algae extract that can be used for the invention include astaxanthins derived from extracts from Haematococcus pluvialis, Haematococcus lacustris, Haematococcus capensis, Haematococcus droebakensis, and Haematococcus zimbabwiensis. However, the invention is not limited thereto.

The Haematococcus algae extract that can be used in the invention may be obtained by crushing cell walls of the above raw material by the method disclosed in JP-A No. 5-68585 or the like, as needed, and extracting with adding an organic solvent such as acetone, ether, chloroform, or an alcohol (ethanol, methanol, etc.) or an extraction solvent such as carbon dioxide in a supercritical state.

Furthermore, widely commercially available Haematococcus algae extracts can be used. For example, ASTOTS-S, ASTOTS-2.5 O, ASTOTS-5 O, and ASTOTS-10 O (all of which are trade names) manufactured by Takeda Shiki Co., Ltd., ASTOTS-S, ASTOTS-2.5 O, ASTOTS-5 O, and ASTOTS-10 O (all of which are trade names), ASTAREAL OIL 50F and ASTAREAL OIL 5F (both of which are trade names) manufactured by Fuji Chemical Industry Co. Ltd., and BIOASTIN SCE7 (trade name) manufactured by Toyo Koso Kagaku Co. Ltd can be used.

The amount of astaxanthins as a pure coloring matter in a Haematococcus algae extract that may be used in the invention is preferably from 0.001% to 50% by mass, more preferably from 0.01 to 25% by mass, from the viewpoint of handling in manufacturing the composition.

In this regard, the Haematococcus algae extract that can be used in the invention may include astaxanthin or an esterified form thereof as a pure coloring matter, similar to a coloring matter as described in JP-A No. 2-49091. In that case, the esterified form thereof is included in a proportion of generally 50% by mole or more, preferably 75% by mole or more, and more preferably 90% by mole or more.

A more detailed description is given in "Astaxanthin-no-Kagaku (Chemistry of Astaxanthin)", 2005 on the Internet (URL: http://www.astaxanthin.co.jp/chemical/basic.htm).

The content of the astaxanthin in the composition of the invention can be appropriately selected according to the form of the composition. For example, in a case where the composition of the invention is applied to a cosmetic preparation, the content is preferably from 0.00001 to 5% by mass, more preferably from 0.0001 to 1% by mass, still more preferably from 0.0001 to 0.1% by mass, based on the total mass (100% by mass) of the cosmetic preparation, from the viewpoint of adjustment of the color of the skin and preference for color.

(B) Lycopene

The astaxanthin-containing composition of the invention contains from 1 to 200 parts by mass based on 100 parts by mass of astaxanthin.

Lycopene (in some cases also referred to as "lycopene") is a carotenoid having chemical formula $C_{40}H_{56}$ (molecular weight: 536.87) and is a red-color colorant having an absorption maximum at 474 nm (acetone), which belongs to carotenes that forms one class of carotenoid.

In lycopene, there are also cis-trans isomers with respect to a conjugated double bond at the center of the molecule. Examples of the isomers include all-trans isomer, 9-cis isomer, and 13-cis isomer. In the invention, any of these isomers may be used.

In the composition of the invention, lycopene may be contained in the form of a lycopene-containing oil or a lycopene-containing paste that has been separated or extracted from natural products containing the lycopene.

In nature, lycopene is contained in tomato, persimmon, watermelon, and pink grapefruit, and the above lycopene-containing oil may be those separated or extracted from these natural products. There are four known product forms: oil, emulsion liquid, paste, and powder forms.

Further, lycopene used in the invention may be the above extracted material, a material that is further purified the extracted as needed, or a synthetic product.

A particularly preferable form of lycopene in the invention is a fat-soluble extract from tomato pulp. The fat-soluble extract from tomato pulp is particularly preferred from the viewpoint of stability in the composition, quality, and productivity.

Here, the term "fat-soluble extract from tomato pulp" means an extract prepared by pulverizing a tomato, centrifuging the pulverized product to obtain a pulp-like solid, and extracting an extract from the solid using an oil solvent.

As for the lycopene as the fat-soluble extract, a widely commercially available tomato extract as the lycopene-containing oil or lycopene-containing paste may be used. Examples thereof include LYC-O-MATO 15% and LYC-O-MATO 6% (trade names, commercially-available from SUNBRIGHT CO., LTD.) and LYCOPENE 18 (trade name, commercially-available from Kyowa Hakko Kirin Co., Ltd.

(C) Tocopherol

The astaxanthin-containing composition of the invention contains from 10000 to 1000000 parts by mass of tocopherol.

The content ratio of lycopene and tocopherol [(B) : (C)] by mass is from 0.5 : 100000 to 10 : 200.

As the tocopherol in the invention, at least one of tocopherol or a derivative thereof is encompassed.

Tocopherol and derivatives thereof are known as naturally occurring vitamin-E homologues, and they occur widely in nature, for example, in green leaf plants, seaweeds, shellfish, fish, higher animals, and humans. Examples of materials having a particularly high content of the vitamin-E homologues include wheat germ oil (150 to 550 mg%, $\alpha$, $\beta$), soybean oil (100 to 300 mg%, $\alpha$, $\gamma$, $\delta$), corn oil (70 to 250 mg%, $\alpha$, $\gamma$), and cotton seed oil (80 to 110 mg%, $\alpha$, $\gamma$). Among animal oils, cod liver oil contains the vitamin-E homologues in a high amount of 26 to 36 mg%, and all of them are $\alpha$-tocopherol. $\beta$-tocopherol is contained in rice bran oil or palm oil.

As a raw material of natural vitamin E, an oil distillate, which is a byproduct produced in the step of purifying plant oil, particularly in the deodorization step, is generally and widely used. Therefore, a deodorized oil distillate of soybean oil (the ratios of $\alpha$, $\gamma$, and $\delta$ are usually 1 to 2 : 5 to 6 : 3), which is most consumed, is a main raw material. The deodorized oil distillate contains steroid, free fatty acid, glyceride and the like as well as Toc. Thus, as a method for separating these components most efficiently and economically, there is used a combination of operations of solvent extraction, saponification, crystallization, molecular distillation, absorption and the like. Examples thereof include a method including: refluxing an oil distillate using sulfuric acid and methanol to change fatty acid to methyl ester having a low-boiling point; subsequently cooling and filtrating away crystallized steroids; and then subjecting the resulting product to molecular distillation to distill and concentrate tocopherol. Alternatively, the method includes a method that the resulting product is directly treated with a porous strongly basic OH type ion exchange resin so as to allow tocopherol to be selectively adsorbed on the resin, and then tocopherol is eluted and concentrated with a weak acid such as acetic acid or boric acid. In the tocopherol adsorbed to the OH type resin, α-tocopherol is first elution-separated by elution with methanol, then γ-(or β-) tocopherol is elution-separated by elution, and δ-tocopherol is eluted with weak acid, whereby homologues thereof can be isolated and purified.

Natural vitamin E is commercially available as an antioxidants for food, such as mixed tocopherol concentrates with various concentrations such as 40%, 60%, and 80%, and δ-tocopherol concentrate (90% content). For the purpose of use in foods, the molecular distillation operation is usually performed aiming at the effects of deliquoring, deodorization, and decolorization, besides the purification in the final step of purification.

Unless otherwise noted, these components are collectively referred to as "tocopherols".

In this regard, details of tocopherol are described in "Vitamin Handbook (1), fat soluble vitamin" (the Vitamin Society of Japan, ed), issued by Kagaku-Dojin Publishing Company, INC.

The group of compounds consisting of tocopherols and derivatives thereof include those differing in the number and position of methyl group on a chroman ring and side-chain saturation or unsaturation. The group includes dl-α-tocopherol, dl-β-tocopherol, dl-γ-tocopherol, dl-δ-tocopherol, acetic acid-dl-α-tocopherol, nicotinic acid-dl-α-tocopherol, linoleic acid-dl-α-tocopherol, succinic acid-dl-α-tocopherol and the like.

Among them, dl-α-tocopherol, dl-β-tocopherol, dl-γ-tocopherol, dl-δ-tocopherol, and mixtures thereof (mixed tocopherols) are more preferred. Acetic esters of these compounds are preferably used as tocopherol derivatives.

(D) Other components

Other components may be included in the astaxanthin-containing composition of the invention within a range that does not inhibit the effects of the invention.

Other components can be appropriately selected according to the form of the composition of the invention, the purpose or the like. Preferable examples of other components include oil agents, functional oil components, emulsifiers, and other additive components.

(Oil agent)

In the astaxanthin-containing composition of the invention, the term "oil agent" means a substance having an HLB of 13 or less or a LogP value of 1.5 or more, but polymers such as thickeners and inorganic substances are excluded from the scope of the oil agent.

Further, the scope of the oil agent in the invention also includes silicone compounds exhibiting the following properties.

Namely, a silicone compound that separates from water when 0.1% of the silicone compound is added to water at 25°C, silicone compounds that aggregates from water when 0.1% of the silicone compound is added to water at 25 °C, and a silicone compound that disperses in water but has cloudy appearance to the oil agent in the invention. In this case, the term "cloudy" means that the absorbance at 650 nm in a sample in which the compound has been added to water is higher by 0.05 or more, then the absorbance before the addition of the compound to water. Further, any silicone compound that satisfies the above property, are included in the scope of the oil agent in the invention regardless of whether the silicone compound is in solid form, in liquid form, or in wax form at normal temperature.

An emulsifier is generally used as an oil agent determined by the HLB value, however the invention is not particularly limited thereto.

Examples of the oil agent determined by the HLB value include polyglyceryl fatty acid esters such as hexaglyceryl monomyristate (HLB value 11), hexaglyceryl monostearate (HLB value 9.0), hexaglyceryl monooleate (HLB value 9.0), decaglyceryl monostearate (HLB value 12.0), decaglyceryl monoisostearate (HLB value 12.0), decaglyceryl monooleate (HLB value 12.0), decaglyceryl distearate (HLB value 9.5), decaglyceryl disisotearate (HLB value 10.0), and diglyceryl stearate (HLB value 12); and polyoxyethylene glyceryl fatty acid esters such as glyceryl monostearate to which 5 mol of polyoxyethylene has been added (HLB value 9.5) (expressed as "POE (5)", the same herein below).

Further, examples thereof may include polyoxyethylene castor oils and hydrogenated castor oils such as POE (20) castor oil (HLB value 10.5), POE (40) castor oil (HLB value 12.5), POE (20) hydrogenated castor oil (HLB value 10.5), POE (30) hydrogenated castor oil (HLB value 11.0); and polyoxyethylene sterols and hydrogenation sterols such as POE (5) phytosterol (HLB value 9.5) and POE (10) phytosterol (HLB value 12.5).

Further, examples thereof may include polyethylene glycol fatty acid esters such as monolaurate to which 10 mol of polyethylene glycol has been added (HLB value 12.5) (expressed as "PEG (10)", same below), PEG (10) monostearate (HLB value 11.0), PEG (10) monooleate (HLB value 11.0), PEG diisostearate (HLB value 9.5); and polyoxyethylene glyceryl isostearates such as PEG (8) glyceryl isostearate (HLB value 10.0), PEG (10) glyceryl isostearate (HLB value 10.0), PEG (15) glyceryl isostearate (HLB value 12.0), and PEG (20) glyceryl isostearate (HLB value 13.0).

In the invention, the term "HLB" means a value calculated by the Kawakami's formula (HLB = 7 + 11.7 log (Mw/Mo), wherein Mw represents a molecular weight of a hydrophilic group, and Mo represents a molecular weight of a hydrophobic group. Further, numerical values of HLB described in catalogues and the like may be used.

As the oil agent determined by the LogP value, a low-molecular compound is generally used. However, the invention is not particularly limited thereto.

Examples of the oil agent determined by the LogP value include ethylhexyl glycerin (LogP = 1.93), methylparaben (LogP = 1.96), ethyl ethylhexanoate (LogP = 2.85), lauric acid (LogP = 4.1), myristic acid (LogP = 4.9), stearic acid (LogP = 6.61), oleic acid (LogP = 6.36), decanoic acid (LogP = 3.27), ethylhexyl methoxycinnamate (LogP = 4.96), and squalane (LogP = 12.5).

The LogP value can be generally calculated based on the molecular structure. In the invention, the value calculated with ChemDraw Pro (CambridgeSoft) is used. However, the invention is not limited thereto.

In a case where the astaxanthin-containing composition of the invention is formed as an oil composition, preferable oil agents used in the invention are not particularly limited as long as their HLB values are 13 or less or their LogP values are 1.5 or more.

In a case where the astaxanthin-containing composition of the invention is formed as an oil composition, a preferable content of the oil agent used in the invention is not particularly limited as long as the effects of the invention are achieved.

In a case where the astaxanthin-containing composition of the invention is formed as an emulsion composition, examples of the preferable oil agent used in the invention include hexaglyceryl monostearate, hexaglyceryl monooleate, decaglyceryl distearate, decaglyceryl diisostearate, polyglyceryl-2 stearate, POE (5) monostearate, POE (5) phytosterol, cetyl ethylhexanoate, and squalane, oleic acid. Further, as an oil agent, polyglyceryl stearate, polyglyceryl oleate, polyglyceryl isostearate, polyoxyethylene stearate, polyoxyethylene isostearate, polyoxyethylene phytosterol, cetyl ethylhexanoate, squalane, and oleic acid.

These oil agents may be used singly or in combination of two or more kinds thereof.

In a case where the astaxanthin-containing composition of the invention is formed as an emulsion composition, the content of oil agent is preferably 12% by mass or less based on the total mass of the composition. The content is more preferably from 0.1 to 12% by mass, still more preferably from 0.3 to 10% by mass.

In the formation of the astaxanthin-containing composition of the invention as an emulsion composition, in a case where the contents of astaxanthin, lycopene, and tocopherol are 100 parts by mass, 1 to 200 parts by mass, and 200 to 5000 parts by mass, respectively, the content of the oil agent is preferably 12% by mass or less based on the total mass of the emulsion composition, from the viewpoint of suppressing the decomposition of lycopene. In this case, the content of the oil agent is more preferably from 0.1 to 12% by mass, still more preferably from 0.3 to 10% by mass, based on the total mass of the emulsion composition.

(Functional oil component)

The functional oil component is not particularly limited as long as it is an oil-soluble component that is not soluble in an aqueous medium, but is soluble in an oil medium. Functional oil components having physical properties for purposes and functionality can be appropriately selected for use. Examples of other oily components include oily components usually used as ultraviolet absorbers, anti-inflammatory agents, moisturizers, hair protecting agents, whitening agents, anti-spot agents, cell activators, emollient agents, keratolytic agents, antistatic agents, fat soluble vitamins (except for the tocopherol), metabolic syndrome improving agents, antihypertensive drugs, sedatives or the like.

Here, the term "functional oil component" means an oil component which is expected to induce a desired physiological effect in a living body to which the functional oil component is applied.

Examples of the functional oil component include natural ceramides, sugar-modified ceramides such as sphingoglycolipid, and ceramides including ceramide analogs; oils and fats such as olive oil, camellia oil, macadamia nuts oil, castor oil, and coconut oil; hydrocarbons such as liquid paraffin, paraffin, vaseline, ceresin, microcrystallin wax, and squalane; waxes such as carnauba wax, candelilla wax, jojoba oil, yellow beeswax, and lanolin; esters such as isopropyl myristate, 2-octyldodecyl myristate, cetyl 2-ethylhexanoate, and diisostearyl malate; fatty acids such as palmitic acid, stearic acid, and isostearic acid; higher alcohols such as cetyl alcohol, stearyl alcohol, isostearyl alcohol, and 2-octyldodecanol; silicone oils such as methylpolysiloxane and methylphenyl polysiloxane; fatty acid esters of glycerin; high-molecular-weight substances; oil-soluble coloring matters such as other carotenoids; and oil-soluble proteins. Further, examples thereof include various kinds of oils derived from plants and oils derived from animals, which are mixtures of such functional oil component.

(Emulsifier)

In a case where the astaxanthin-containing composition of the invention is formed as an emulsion composition, the composition preferably includes an emulsifier.

The emulsifier that can be used for the invention may be any of anionic surfactants, cationic surfactants, amphoteric surfactants, and nonionic surfactants.

From the viewpoint of emulsifying power, HLB of the emulsifier in the invention is preferably 10 or more, more preferably 12 or more. When HLB is too low, sometimes the emulsifying power may become insufficient. In this regard, from the viewpoint of foam-inhibiting effect, an emulsifier having HLB of from 5 to less than 10 may be used in combination.

Here, the HLB can be calculated by the Kawakami's formula.

Further, the numerical values of HLB described in catalogues and the like may be employed.

Further, as is clear from the Kawakami's formula, emulsifiers having desired HLB values can be obtained by utilizing the additive property of HLB.

The content of the emulsifier generally varies depending on the form of the composition. In the case of the emulsion composition, the content is preferably from 0.5 to 30% by mass, more preferably from 1 to 20% by mass, still more preferably from 2 to 15% by mass, with respect to the total mass of the composition. In the case of the powder composition, the content is preferably from 0.1 to 50% by mass, more preferably from 5 to 45% by mass, still more preferably from 10 to 30% by mass, with respect to the total mass of the composition. The above range is preferred, considering that a content is within the range easily reduces interfacial tension between oil phase/poor solvent, so that it hardly causes a problem such as occurrence of serious foaming in the dispersion composition without adding excessive amounts of the emulsifier.

Further, the total mass of the emulsifier, in either of the powder composition or the emulsion composition, is preferably in a range of 0.1 to 10 times the total mass of the oil components. From the viewpoints of decreasing the size of dispersion particles and supressing foaming, a range of 0.5 to 8 times the total mass of the oil components is more preferable, and a range of 0.8 to 5 times the total mass of the oil components is particularly preferable. These ranges make it possible to provide good dispersion stability of the composition.

Among the emulsifiers, nonionic surfactants are preferred from the viewpoint of low-irritating property and low-environmental impact. Examples of the nonionic surfactants include sucrose fatty acid ester, polyglyceryl fatty acid ester, organic acid monoglyceride, propylene glycol fatty acid ester, polyglyceryl condensed ricinoleate, sorbitan fatty acid ester, and polyoxyethylene sorbitan fatty acid ester.

The emulsifier in the invention may include a phospholipid such as lecithin. Lecithin used in the invention contains a glycerin skeleton, a fatty acid residue, and a phosphoric acid residue as essential constituent components, to which a base, a polyhydric alcohol, or the like is bonded, and is also referred to as a phospholipid. The phospholipid has a hydrophilic group and a hydrophobic group in the molecule thereof, and therefore has been widely used as an emulsifier in the fields of foods, pharmaceutical products, and cosmetic products.

Lecithins having a purity of 60% or more are used for industrial use, and such a lecithin can be used also in the invention. A preferable lecithin is one generally referred to as a high-purity lecithin, which has preferably a purity of 80% or more, and more preferably 90% or more from the viewpoint of formation of fine oil droplet particle size and stability of functional oily component.

Examples of the phospholipid may include various conventionally known products extracted and separated from living bodies of plants, animals, and microorganisms.

Specific examples of the phospholipid include various kinds of lecithins derived from plants such as soybean, corn, peanut, rapeseed, or wheat; egg yolk; animals such as cattle; and microorganisms such as Escherichia coli.

Examples of compound name of lecithins include glycerolecithins such as phosphatidic acid, phosphatidylglycerin, phosphatidylinositol, phosphatidylethanolamine, phosphatidylmethylethanolamine, phosphatidylcholine, phosphatidylserine, bisphosphatidic acid, and diphosphatidylglycerin (cardiolipin); and sphingolecithins such as sphingomyelin.

In addition, in the invention, besides the high-purity lecithin as described above, a hydrogenated lecithin, an enzyme-decomposed lecithin, an enzyme-decomposed hydrogenated lecithin, a hydroxylecithin, or the like may be used. These lecithins that can be used in the invention may be used singly or in the form of a mixture of a plurality of kinds of lecithins.

(Other additive components)

Besides the above-described components, additive components that are ordinarily used in the field of foods, cosmetics and the like may be appropriately included in the composition of the invention in accordance with the form of the composition. The additive components may be included in the composition of the invention as an oil-soluble or water-soluble additive component in accordance with properties of the additive components.

For example, various kinds of additive components disclosed in References 1 to 6 below may be freely used as needed.

Reference 1: "Marketing of Materials of Functional Cosmetics", 2009, CMC Publishing Co., LTD.

Reference 2: "Keshouhin Seibun Yougo Jiten (Dictionary of Cosmetic Ingredient Terms), Kazunari Suzuki, 2008, Chuou Shoin.

Reference 3: "Selection and Formula examples of Additives in the Drug and Cosmetic Fields and Application for Updating the Regulation", TECHNICAL INFORMATION INSTITUTE CO., LTD

Reference 4: "Keshouhin Yuhko Seibun Handbook (Handbook of Active Components for Cosmetics)", Tadamasa Shimokawa, Chuou Shoin

Reference 5: "Zenseibun Hyoji ni Taioshita Keshohin Seibun Gaido (Guide of Cosmetic Components Responding to Labeling of All Ingredients), 4th ed.", Masaharu Yuasa, FRAGRANCE JOURNAL LTD.

Reference 6: "Japanese Cosmetic Labeling Name Dictionary, 2nd ed., Appendix", Japan Cosmetic Industry Association, YAKUJI NIPPO LIMITED.

Examples of other additive components include polyhydric alcohols such as glycerin and 1,3-butylene glycol; monosaccharides or polysaccharides such as glucose, fructose, lactose, maltose, sucrose, pectin, κ-carrageenen, locust bean gum, guar gum, hydroxypropyl guar gum, xanthan gum, karaya gum, tamarind seed polysaccharide, gum arabic, tragacanth gum, hyaluronic acid, sodium hyaluronate, sodium chondroitin sulfate, and dextrin; sugar alcohols such as sorbitol, mannitol, maltitol, lactose, maltothreitol, and xylitol; inorganic salts such as sodium chloride and sodium sulfate; proteins having a molecular weight of more than 5000 such as casein, albumin, methylated collagen, hydrolyzed collagen, water-soluble collagen, and gelatin; amino acids and derivatives thereof such as glycine, alanine, valine, leucine, isoleucine, serine, threonine, aspartic acid, glutamic acid, cystine, methionine, lysine, hydroxylysine, arginine, histidine, phenylalanine, thyrosin, tryptophan, proline, hydroxyproline, and acetylhydroxyproline; synthetic polymers such as carboxyvinyl polymer, sodium polyacrylate, polyvinyl alcohol, polyethylene glycol and ethyleneoxide-propyleneoxide block copolymer; water-soluble cellulose derivatives such as hydroxyethyl cellulose and methyl cellulose; flavonoids (catechin, anthocyanin, flavone, isoflavone, fravane, fravanone, rutin); ascorbic acids or derivatives thereof (ascorbic acid, sodium ascorbate, potassium ascorbate, calcium ascorbate, L-ascorbic acid phosphate, magnesium ascorbyl phosphate, sodium ascorbyl phosphate, ascorbyl sulfate, disodium ascorbyl sulfate, ascorbyl-2-glucoside, etc.); tocotrienols and derivatives thereof; phenolic acids (chlorogenic acid, ellagic acid, gallic acid, propyl gallate); lignans, curcumins, coumarins; and hydroxy stilbenes including pterostilbene. These components may be contained in accordance with their functions, for example, as a functional component, an excipient, a viscosity modifier, a radical scavenger and the like.

In addition, in the invention, other additives that are usually used for the intended use, for example, various kinds of medically effective components, a pH adjuster, a pH buffer, a ultraviolet absorber, an antiseptic agent, perfume, and a colorant, may be used in combination.

(Purposes of use)

The astaxanthin-containing composition of the invention is excellent in storage stability, and thus a desired effect such as antioxidant capacity of astaxanthin is expected to be improved. Therefore, it can be used for various purposes of use, such as cosmetics preparations, foods, and pharmaceutical products.

[Cosmetic preparation]

A suitable application embodiment the astaxanthin-containing composition of the invention is a cosmetic preparation. Examples of the cosmetic preparation may include lotion, beauty essence, milky lotion, cream, facial mask, facial pack, shampoo cosmetics, fragrance cosmetics, liquid body cleaning preparations, UV care cosmetics, deodorant cosmetics, cosmetics for oral health.

The astaxanthin-containing composition of the invention can be suitably manufactured by the manufacturing method to be described in detail below.

[Method of manufacturing astaxanthin-containing composition] The astaxanthin-containing composition of the invention is prepared by combining (A) 100 parts by mass of astaxanthin, (B) 1 to 200 parts by mass oflycopene, and (C) 10000 to 1000000 parts by mass of tocopherol and optional components as required.

In this regard, details of the components used to prepare the astaxanthin-containing composition of the invention are as described for the essential components and arbitrary components in the astaxanthin-containing composition of the invention, and preferred ranges thereof are also the same.

In preparation of the astaxanthin-containing composition of the invention, the method for combining astaxanthin, lycopene, tocopherol, and the optional components that are included as required is not particularly limited.

In a case where the astaxanthin-containing composition of the invention is an oil composition, for example, predetermined amounts of astaxanthin, lycopene, tocopherol, and optional components may be mixed and stirred at normal temperature (25°C) using an optional stirring means.

In a case where the astaxanthin-containing composition of the invention is an emulsion composition, an oil phase including predetermined amounts of astaxanthin, lycopene, tocopherol, and the optional components may be prepared by the manufacturing method of the invention, and the oil phase is mixed with an aqueous phase including an aqueous phase component to obtain an emulsion composition.

In a case where the emulsion composition is obtained by the manufacturing method of the invention, the fat-soluble extract derived from tomato pulp is particularly preferred as lycopene to be used. The lycopene can be stably emulsified by using the lycopene of the fat-soluble extract derived from tomato pulp.

The ratio (mass) of an oil phase and an aqueous phase in the emulsification is not particularly limited. However, the ratio (% by mass) of oil phase/aqueous phase is preferably from 0.1/99.9 to 50/50, more preferably from 0.5/99.5 to 30/70, and still more preferably from 1/99 to 20/80.

Decrease in the content of active components is prevented by setting the ratio of oil phase/aqueous phase to 0.1/99.9 or more, which results in a favorable trend that a practical problem of the emulsion composition is not caused. Further, by setting the ratio of oil phase/aqueous phase to 50/50 or less, decrease in concentration of the emulsifier is prevented and become a favorable trendency that emulsion stability of the emulsion composition is not deteriorated.

The emulsification may be performed by one step-operation of the emulsification. However, it is preferable to perform two or more step-operations of the emulsification, from the viewpoint of obtaining uniform and fine emulsified particles.

Specifically, it is especially preferable to use a combination of two or more kinds of emulsification apparatuses in a manner such that emulsification is performed by way of a high-pressure homogenizer or the like in addition to the one step-operation of the emulsification in which emulsification is performed using an ordinary emulsification apparatus using a shearing force (for example, stirrer, impeller agitation, homo-mixer, continuous-flow type shearing machine). The use of a high-pressure homogenizer allows the emulsion to have liquid droplets of uniform fine particles. Further, plural operations may be additionally performed in order to make the particle diameter of the liquid droplets more uniform.

As an emulsification means, any of generally known emulsification techniques such as a spontaneous emulsification method, an interfacial chemical emulsification method, an electric emulsification method, a capillary emulsification method, a mechanical emulsification method, a supersonic emulsification method can be used.

As a useful method for making the emulsified particles finer, an interfacial chemical emulsification method such as a PIT emulsification method or a gel emulsification method is known. This method has an advantage in that consumption energy is low, and therefore the method is suitable in a case of finely emulsifying a material that is easy to deteriorate by heat.

Further, as a generally-used emulsification method, a method of using a mechanical force is applied, that is, the method of tearing apart oil droplets by applying a shearing force thereto from the outside. The most-general force of the mechanical force is a high-speed and high-shearing mixer. As such a mixer, mixers that are referred to as a homo-mixer, a disper mixer or an ultra-mixer are commercially available.

As another mechanical emulsification apparatus that is useful for refining particles, a high-pressure homogenizer is available and various kinds of apparatuses are commercially available. The high-pressure homogenizer is capable of applying greater shearing force than a stirring method. Accordingly, even if the amount of an emulsifier is relatively small, refining particles can be realized.

High-pressure homogenizer is generally divided into a chamber type high-pressure homogenizer having a fixed throttling section and a homogenizing valve type high-pressure homogenizer in which the opening degree of throttle is controlled.

Examples of the chamber type high-pressure homogenizer include MICROFLUIDIZER (manufactured by Microfluidics Corporation), NANOMIZER (manufactured by Yoshida Kikai Co., Ltd.) and ALTIMIZER (manufactured by Sugino Machine Limited).

Examples of the homogenizing valve type high-pressure homogenizer include Gaulin-type homogenizer (manufactured by APV Company), Rannie-type homogenizer (manufactured by Rannie Company), HIGH-PRESSURE HOMOGENIZER (manufactured by GEA Niro Soavi), HOMOGENIZER (manufactured by SANNWA MACHINERY TRADING CO., LTD.), HIGH-PRESSURE HOMOGENIZER (manufactured by IZUMI FOOD MACHINERY CO., LTD.), and ULTRAHIGH-PRESSURE HOMOGENIZER (manufactured by IKA Corporation).

There is an ultrasonic homogenizer as a dispersing apparatus having a relatively good energy efficiency and having a simple structure. Examples of a high-power ultrasonic homogenizer that can be produced include ultrasonic homogenizer US-600, US-1200T, RUS-1200T, and MUS-1200T (all of which are trade names, manufactured by NISSEI Corporation), ultrasonic processor UIP 2000, UIP 4000, UIP 8000, and UIP 16000 (all of which are trade names, manufactured by Hielscher). These high-power ultrasonic homogenizers are used at a frequency of 25 kHz or less, preferably at a frequency of from 15 to 20 kHz.

Further, as another known emulsification means, a method of using a static mixer, a micro channel, a macro mixer, and a membrane emulsification apparatus and the like that do not have an extraneous agitation section and needs only low energy is also useful. e.

The temperature condition in emulsification dispersion in the invention is not particularly limited. From the viewpoint of stability of functional oil components, the range of from 10 to 100°C is preferable. A favorable range can be appropriately selected depending on such as a melting point of the functional oil component to be handled.

Further, in a case of using a high-pressure homogenizer in the invention, the processing is preferably performed at a pressure of from 50 MPa or more, more preferably at a pressure of from 50 to 280 MPa, still more preferably at a pressure of from 100 to 280 MPa.

Further, from the viewpoint of keeping particle diameter of the dispersion particles, it is preferable that an emulsified liquid that is an emulsion dispersed composition is cooled through some sort of cooling machine within 30 seconds after the emulsified liquid has passed through a chamber, preferable within 3 seconds immediately.

The manufacturing method of the invention may include drying the obtained emulsion composition to obtain a powder composition. This method makes it possible to obtain a astaxanthin-containing composition as a powder composition. The astaxanthin-containing composition as a powder composition has storage stability, and the emulsion composition in the form that the powder composition has been re-dissolved in an aqueous medium also has storage stability besides powderization form.

As for the drying means used in manufacturing the powder composition, a known drying means may be used. Examples of the drying means include natural drying, heat drying, hot air drying, high-frequency drying, ultrasonic drying, reduced-pressure drying, vacuum drying, freeze drying, and spray drying. These means may be used singly, or in combination of two or more kinds thereof.

In the invention, reduced-pressure drying, vacuum drying, freeze drying, and spray drying are preferred, because functional materials that are relatively weak against heat are often contained. Further, a method of conducting vacuum (reduced-pressure) drying while keeping the temperature in a range of 0°C or lower and freezing temperature or higher, which is one of the vacuum drying methods, is also preferred.

In a case of vacuum drying or reduced-pressure drying, the drying in which concentration is repeated while gradually increasing the degree of reduced pressure in order to avoid scatter of the liquid due to bumping is also preferable.

In the invention, the freeze drying in which ice is sublimated from a material in a frozen state to remove water is preferred. The freeze dry method has a great advantage such that since the drying process is usually performed at 0°C or lower, ordinarily at a range of about -20°C to -50°C, heat denaturation of the material is not caused, and in the course of water recovery, taste, color, nutritional value, shape, texture and the like are easy to reconstitute to their state before drying.

Examples of the commercially available freeze dryer include FREEZE DRYER VD-800F (trade name, manufactured by TAITEC Corporation), FLEXI-DRY MP (trade name, manufactured by FTS SYSTEMS INC.), DURATOP · DURASTOP (trade name, manufactured by FTS SYSTEMS INC.), TAKARA FREEZE-DRYER MODEL A (trade name, manufactured by TAKARA ATM), DESKTOP FREEZE-DRYER FD-1000 (trade name, manufactured by TOKYO RIKAKIKAI CO., LTD.), VACCUM FREEZE-DRYER FD-550 (trade name, manufactured by TOKYO RIKAKIKAI CO., LTD.), and VACCUM FREEZE-DRYER (trade name, manufactured by TAKARA SEISAKUSYO). However, the invention is not limited thereto.

Further, in the invention, a spray-drying method is particularly preferred as a drying means from the viewpoint of satisfying both of production efficiency and quality. The spray drying is a sort of convective-hot air drying. The liquid composition is sprayed as fine particles of several $100\mu m$ or less in a hot air and resultantly drops in a tower while being dried, whereby the composition is collected as a solid powder thereof. Though the material is temporarily exposed to hot air, increase of temperature of the material does not become too high because of very short exposure time and vapor latent heat, and therefore heat denaturation of the material is not easy to be caused and a change due to reconstitution by water is small as is the case with freeze drying. In a case of a material that is very weak against heat, it is also possible to feed cold air instead of hot air. In this case, milder drying can be favorably realized, though the drying performance is reduced.

Examples of the commercially available spray dryer include a spray dryer SPRAY DRYER SD-1000 (trade name, manufactured by TOKYO RIKAKIKAI CO., LTD.), SPRAY DRYER L-8i (trade name, manufactured by OHKAWARA KAKOHKI Co., LTD.), CLOSED SPRAY DRYER CL-12 (trade name, manufactured by OHKAWARA KAKOHKI Co., LTD.), SPRAY DRYER ADL 310 (trade name, manufactured by YAMATO SCIENTIFIC CO., LTD.), MINISPRAY DRYER B-290 (trade name, manufactured by BUCHI Labortechnik AG), PJ-MINIMAX (trade name, manufactured by Powdering Japan) and PHARMASD (trade name, manufactured by GEA Niro). However, the invention is not limited thereto.

Further, producing granular particles exerting excellent handling property at the same time as drying by using an apparatus by which both drying and granulation can be performed at the same time, such as fluid-bed granulation dryer MP-01 (trade name, manufactured by POWREX CORPORATION) and spray dryer with a built-in fluid-bed FSD (trade name, manufactured by GEA Niro).

The average particle diameter of the emulsion composition obtained by the manufacturing method of the invention or the average particle diameter of the powder composition obtained by powderization of the emulsion composition means a particle diameter of the emulsified particles (oil droplets) in the emulsion composition in a case of the emulsion composition, and means a particle diameter of the emulsified particles (oil droplets) in a 1% by mass aqueous solution (at the time of re-dissolution) in a case of the powder composition.

The particle diameter of the emulsified particles can be measured using a commercially-available particle diameter distribution measuring apparatus or the like. As for the particle diameter distribution measuring method, optical microscopy, laser confocal microscopy, electron microscopy, atomic force microscopy, static light-scattering method, laser diffractometry, dynamic light-scattering method, centrifugal precipitation method, electric pulse measuring technique, chromatographic method and ultrasonic attenuation method are known. Apparatuses in accordance with their principles are commercially available.

The dynamic light-scattering method is preferable in the particle diameter measurement of the emulsified particles in the invention. Examples of the commercially-available measuring apparatus using the dynamic light-scattering method include NANOTRAC UPA (trade name, manufactured by Nikkiso Co., Ltd.), dynamic light-scattering method particle diameter distribution measuring apparatus LB-550 (trade name, manufactured by HORIBA, Ltd.), and concentrated system particle diameter analyzer FPAR-1000 (trade name, manufactured by Otsuka Electronics Co., Ltd.). However, as the particle diameter in the invention, a value obtained by measurement at 25°C using the particle diameter analyzer FPAR-1000 (trade name, manufactured by Otsuka Electronics Co., Ltd.) is adapted.

Further, the particle diameter of emulsified particles may be adjusted by factors such as agitation conditions in the manufacturing method (shearing force, temperature, pressure) and ratio of oil phase and aqueous phase, besides the components of the composition.

The particle diameter of the emulsified particles in the emulsion composition is preferably from 50 nm to 300 nm from the viewpoint of transparency and absorbing property. The particle diameter is more preferably from 50 nm to 200 nm, most preferably from 50 nm to 150 nm from the viewpoint of transparency.

EXAMPLES

Hereinafter, Examples of the invention will be described, however, the invention is not limited to the Examples. Unless otherwise noted, the numerical numbers expressed by "part" and "%" which show the amounts of components in compositions in the Examples and Comparative examples are based on mass standard.

[Examples 1 to 8, Comparative examples 1 to 6]

<Stability of astaxanthin-containing composition prepared as oil composition>

(Preparation of astaxanthin-containing composition)

Astaxanthin, lycopene, tocopherol, and COCONAD MT were weighed so that the content ratios (%) of astaxanthin, lycopene, and tocopherol based on the total mass of the composition had the ratios shown in Table 1 below. These components was stirred at room temperature (25°C) for 1 hour to prepare 200 g of each of the astaxanthin-containing compositions of Examples 1 to 8 and Comparative examples 1 to 6 (oil compositions).

In this regard, when the content of astaxanthin in the compositions of Examples 1 to 8 and Comparative examples 1 to 6 is 100 parts by mass, the mass ratios of astaxanthin, lycopene, and tocopherol are described together in Table 1. However, as for Comparative example 2, the ratio is indicated as the mass ratio of lycopene and tocopherol when an equivalent amount to that of astaxanthin in the composition of Example 1 is 100 parts by mass.

Details of the respective components used to prepare the astaxanthin-containing compositions of Examples 1 to 8 and Comparative examples 1 to 6 are as follows:

- Astaxanthin (Haematococcus algae pigment, astaxanthin content: 20% by mass, trade name: ASTOTS-S, manufactured by Takeda Shiki Co., Ltd.)
- Lycopene (trade name: LYCOPENE 18, lycopene paste, manufactured by Kyowa Wellness Co., Ltd., lycopene concentration: 18%, molecular weight: 537.0)
- Tocopherol (trade name: RIKEN E OIL 800, Mix tocopherol, manufactured by RIKEN VITAMIN CO., LTD)
- COCONAD MT (trade name, tri(caprylic acid/caproic acid)glyceryl, HLB = 1, manufactured by Kao Corporation)

(Stability of astaxanthin-containing composition prepared as oil composition)

The storage stability of the obtained astaxanthin-containing compositions of the examples and comparative examples was evaluated as shown in Evaluations (1) and (2). The evaluation method and evaluation criteria are as described below.

<Evaluation (1): residual-ratios of astaxanthin and lycopene>

The compositions of the examples shown in Table 1 were prepared. Thereafter, the dilution ratio with a COCONAD MT liquid in each of the compositions of the examples was calculated so that the $\lambda$max absorbance in the vicinity of 480 nm of the transmission UV spectrum was 2.0. The astaxanthin-containing compositions were diluted to prepare samples for evaluation. Astaxanthin or lycopene was diluted with the COCONAD MT liquid at the same concentration as that of astaxanthin or lycopene contained in each of the compositions of the examples, and a sample of astaxanthin alone and a sample of lycopene alone were prepared.

As for the compositions of the examples, the sample of astaxanthin alone, and the sample of lycopene alone, the absorbance at 480 nm immediately after preparation and after storage at 50°C for 1 month following preparation was measured.

A UV-VIS SPECTROPHOTOMETER UV-2550 (trade name, manufactured by Shimadzu Corporation) was used to measure absorbance. The evaluation results are shown in Table 1.

<Evaluation criteria>

The residual ratios of astaxanthin and lycopene were evaluated according to the following evaluation criteria:

Residual ratio of astaxanthin

> A: 95% or more
> B: 91% or more and less than 95%
> C: less than 91%

Residual ratio of lycopene

> s: 90% or more
> a: 81 % or more and less than 90%
> b: less than 81 %

<Evaluation (2): precipitate>

50 g of each of the compositions of the examples shown in Table 1 was aliquoted to each vial bottle, and samples for evaluation were prepared. After the preparation, the samples were stored at 50°C for 1 month. In each of the stored samples for evaluation, a portion of the surface, 4 portions of the edge of central part and a portion of the central part were collected. The presence or absence of precipitates (crystals derived from lycopene) was visually observed using an optical microscope (magnification, ×10).

The sample in which no precipitate was observed was evaluated "A" and the sample in which the precipitates were observed was evaluated as "B". The evaluation results are shown in Table 1.

[Table 1]

| | (A) Astaxanthin (%) | (B) Lycopene (%) | (C) Tocopherol (%) | Mass ratios of (A), (B), and (C) (part by mass) | Evaluation of storage stability (50°C1M) | | | | |
| --- | --- | --- | --- | --- | --- | --- | --- | --- | --- |
| | | | | | Residual ratio | | | | Precipitation |
| | | | | | Astaxanthin | Evaluation | Lycopene | Evaluation | |
| Example 1 | 0.001 | 0.01 | 0.5 | 100 : 1000 : 50000 | 95% | A | 82% | a | A |
| Example 2 | 0.001 | 0.002 | 0.5 | 100 : 200 : 50000 | 95% | A | 88% | a | A |
| Example 3 | 0.001 | 0.001 | 0.5 | 100 : 100 : 50000 | 97% | A | 89% | a | A |
| Example 4 | 0.001 | 0.0005 | 0.5 | 100 : 50 : 50000 | 97% | A | 92% | s | A |
| Example 5 | 0.001 | 0.0005 | 0.1 | 100 : 50 : 10000 | 97% | A | 90% | s | A |
| Example 6 | 0.001 | 0.0005 | 1 | 100 : 50 : 100000 | 98% | A | 93% | s | A |
| Example 7 | 0.001 | 0.0001 | 0.5 | 100 : 10 : 50000 | 97% | A | 93% | s | A |
| Example 8 | 0.001 | 0.00001 | 0.5 | 100 : 1 : 50000 | 98% | A | 92% | s | A |
| Comparative example 1 | 0.001 | - | 0.5 | 100 : - : 50000 | 90% | C | - | - | A |
| Comparative example 2 | - | 0.001 | 0.5 | - : 100 : 50000 | - | - | 68% | b | B |
| Comparative example 3 | 0.001 | 0.001 | - | 100 : 100 : - | 92% | B | 70% | b | A |
| Comparative example 4 | 0.1 | 0.1 | 0.1 | 100 : 100 : 100 | 93% | B | 72% | b | A |
| Comparative example 5 | 0.001 | 0.001 | 0.001 | 100 : 100 : 100 | 92% | B | 75% | b | A |
| Comparative example 6 | 0.001 | 0.00005 | 0.0015 | 100 : 5 : 150 | 92% | B | 77% | b | A |

Specifically, the residual ratio (%) of astaxanthin and lycopene in Table 1 are calculated by Equations (a) and (b) below.

[Formula 1]

$$(\text{Formula a}) : \text{Residual ratio (\%) of astaxanthin} = \frac{\text{after storage (absorbance of sample for evaluation) - (absorbance of sample of lycopene alone)}}{\text{Immediately after preparation (absorbance of sample for evaluation) - (absorbance of sample of lycopene alone)}} \times 100$$

$$(\text{Formula b}) : \text{Residual ratio (\%) of lycopene} = \frac{\text{after storage (absorbance of sample for evaluation) - (absorbance of sample of astaxanthin alone)}}{\text{Immediately after preparation (absorbance of sample for evaluation) - (absorbance of sample of astaxanthin alone)}} \times 100$$

In Table 2, a calculation method of residual ratios of astaxanthin and lycopene in Example 4 is described in detail.

[Table 2]

| | | Storage conditions | Absorbance |
|---|---|---|---|
| Sample for evaluation | Example 4 | Immediately after preparation | 1.630 |
| Sample for evaluation | Example 4 | After storage at 50°C for 1 month | 1.558 |
| Sample of one component alone | Astaxanthin | Immediately after preparation | 0.946 |
| Sample of one component alone | Astaxanthin | After storage at 50°C for 1 month | 0.930 |
| Sample of one component alone | Lycopene | Immediately after preparation | 0.749 |
| Sample of one component alone | Lycopene | After storage at 50°C for 1 month | 0.700 |

$$(\text{Formula a}) \qquad \text{Residual ratio of astaxanthin (\%)} = \{(1.558 - 0.700)/(1.630 - 0.749)\} \times 100 = 97\,(\%)$$

$$(\text{Formula b}) \qquad \text{Residual ratio of lycopene (\%)} = \{(1.558 - 0.930)/(1.630 - 0.946)\} \times 100 = 92\,(\%)$$

As shown in Table 1, all the astaxanthin-containing compositions of the examples have high residual ratios (%) of astaxanthin and lycopene, do not generate precipitates, and were excellent in storage stability is found.

In each of the examples, the astaxanthin-containing composition as an oil composition was prepared. However, an emulsion composition can be prepared by using the oil composition as an oil phase, emulsifying the composition together with an aqueous phase including an arbitrary aqueous phase component such as glycerin. The emulsion compositions also show the similar excellent storage stability as that of the oil compositions prepared in the examples.

Further, the astaxanthin-containing composition of each of the examples has excellent storage stability. Thus, the astaxanthin-containing composition or an emulsion composition including the astaxanthin-containing composition as an oil phase component is used as a cosmetic preparation so that a cosmetic preparation excellent in storage stability can be obtained.

[Examples 9 to 16, Comparative example 7]

<Stability 1 of astaxanthin-containing composition prepared as emulsion composition>

(Preparation of milky lotion of astaxanthin-containing composition)

Milky lotions having the composition shown in Table 3 below were prepared.

Astaxanthin, lycopene, tocopherol, and COCONAD MT were weighed so as to have the content ratios (%) shown in Tables 3 and 4.

These components were stirred at room temperature (25°C) for 1 hour to prepare a mixture of astaxanthin, lycopene, tocopherol, and COCONAD MT.

The oil agent shown in Table 3 was added to the prepared mixture to prepare an oil phase. The oil phase was stored at 60°C.

Subsequently, 1,3-butylene glycol, glycerin, and water were mixed to prepare an aqueous phase. The aqueous phase was stored at 60°C.

The oil phase was mixed and emulsified with the aqueous phase using a homomixer at 60°C, and milky lotions of the astaxanthin-containing compositions of Examples 9 to 16 were prepared so as to have a particle size of 0.5 to 2.0 $\mu$m.

In this regard, each of the emulsion compositions was 20-fold diluted with pure water, and the particle size was determined as a median size (d = 50) using a particle diameter analyzer FPAR-1000 (trade name, manufactured by Otsuka Electronics Co., Ltd.).

Details of the respective components used to prepare the milky lotions including the astaxanthin-containing compositions of Examples 9 to 16 and Comparative example 7 are as follows:

- Bis-ethoxyglycol cyclohexane 1,4-dicarboxylate (trade name, NEOSOLUE-AQULIO, LogP = 0.01, manufactured by Nippon Fine Chemical Co., Ltd.)
- Isotridecyl isononanoate (trade name, EMALEXINTD-139, HLB = 2.0, manufactured by Nihon-Emulsion Co., Ltd.)
- Polyoxyethylene cetyl ether (trade name, BC-5.5, HLB = 10.5, manufactured by Nippon Chemicals Sales Co., Ltd.)
- Polyoxyethylene sorbitan monooleate (trade name, LEODOLE TW-0106V, HLB = 10, manufactured by Kao Corporation)
- Ethylhexyl glycerin (trade name, SENSIYAAC50JP, LogP = 1.93, manufactured by Seiwa Kasei Co., Ltd.)
- Sucrose stearate (trade name, SURFHOPE SE COSME C-1805, HLB = 5, manufactured by Mitsubishi-Kagaku Foods Corporation)

(Stability of astaxanthin-containing composition prepared as emulsion composition)

The storage stability of the obtained milky lotions including the astaxanthin-containing compositions of the examples and comparative examples were evaluated as shown in Evaluation (3). The evaluation method and evaluation criteria are as described below.

<Evaluation (3): residual ratios of astaxanthin and lycopene>

Immediately after preparation and after storage at 50°C for 7 days, the milky lotions of Table 4 were measured by high-speed column chromatography. The measurement result immediately after preparation was defined as 100%, and the residual ratios of astaxanthin and lycopene after storage at 50°C for 7 days was calculated. The evaluation results are shown in Table 4.

<Evaluation criteria>

The residual ratios of astaxanthin and lycopene were evaluated according to the following evaluation criteria:

Residual ratio of astaxanthin

A: 90% or more

B: 76% or more and less than 90%

C: less than 76%

Residual ratio of lycopene

s: 80% or more

a: 51 % or more and less than 80%

b: 31% or more and less than 50%

c: less than 31 %

[Table 3]

| Oil phase | | Examples 10, 12 to 16 Comparative example 7 | Example 9 | Example 11 |
|---|---|---|---|---|
| | Astaxanthin, lycopene, and tocopherol blended at the ratios shown in Table 4 | 1.0 | 1.0 | 1.0 |
| | COCONAD MT | | | |
| Oil agent | Bis-ethoxyglycol cyclohexane 1,4-dicarboxylate | 1.0 | 2.0 | 2.0 |
| | Ethylenecetyl ether isononanoate | 2.0 | 4.0 | 4.0 |
| | Polyoxyethylene cetyl ether | 2.0 | 4.0 | 4.0 |
| | Polyoxyethylene Sorbitan monooleate | 2.0 | 4.0 | 4.0 |
| | Ethylhexyl glycerin | 0.5 | 1.0 | 1.0 |
| | Sucrose stearate | 0.1 | 0.2 | 0.05 |
| | Total of oil agents | 7.6 | 15.2 | 3.8 |
| Aqueous phase | | | | |
| 1,3-butylene glycol | | 80 | 80 | 80 |
| Glycerin | | 20 | 20 | |
| Water | | Balance | Balance | Balance |
| Total | | 100 | 100 | 100 |
| (% by mass) | | | | |

[Table 4]

| | (A) Astaxanthin (%) | (B) Lycopene (%) | (C) Tocopherol (%) | Mass ratios of (A), (B), and (C) (part by mass) | Oil agent | Evaluation of storage stability (50°C7d) | | | |
|---|---|---|---|---|---|---|---|---|---|
| | | | | | | Residual ratio | | | |
| | | | | | | Astaxanthin | Evaluation | Lycopene | Evaluation |
| Example 9 | 0.005 | 0.0005 | 0.750 | 100 : 10 : 15000 | 15.2 | 94% | A | 82% | a |
| Example 10 | 0.005 | 0.0005 | 0.750 | 100 : 10 : 15000 | 7.6 | 93% | A | 45% | b |
| Example 11 | 0.005 | 0.0005 | 0.750 | 100 : 10 : 15000 | 3.8 | 91% | A | 34% | b |
| Example 12 | 0.005 | 0.0005 | 0.350 | 100 : 10 : 7000 | 7.6 | 94% | A | 59% | a |
| Example 13 | 0.005 | 0.0025 | 0.075 | 100 : 50 : 1500 | 7.6 | 94% | A | 83% | s |
| Example 14 | 0.005 | 0.0005 | 0.075 | 100 : 10 : 1500 | 7.6 | 96% | A | 83% | s |
| Example 15 | 0.005 | 0.0005 | 0.035 | 100 : 10 : 700 | 7.6 | 95% | A | 83% | s |
| Example 16 | 0.005 | 0.0005 | 0.010 | 100 : 10 : 200 | 7.6 | 96% | A | 85% | s |
| Comparative example 7 | 0.005 | 0.0005 | - | 100 : 10 : - | 7.6 | 86% | B | 23% | c |

In all the milky lotions in Examples 9 to 16 of Table 4, it is found that the residual ratio of astaxanthin is excellent. In a case where the blending amounts of tocopherol are shown in Examples 13 to 16, it is found out that, surprisingly, the residual ratio of lycopene is so excellent.

[Examples 17 and 18]

<Stability 2 of astaxanthin-containing composition prepared as emulsion composition>

(Preparation of milky lotion of astaxanthin-containing composition)

In order to improve the residual ratio of lycopene in Example 10 of Table 4, the oil agents to be added to the oil phase were examined.
In Examples 17 and 18, bis-ethoxyglycol cyclohexane 1,4-dicarboxylate of the oil agent in Table 3 was changed. In Example 17, the bis-ethoxyglycol cyclohexane 1,4-dicarboxylate was changed to squalane. In Example 18, bis-ethoxyglycol cyclohexane 1,4-dicarboxylate was changed to cetyl ethylhexanoate. The blending amount and other components were not changed and each milky lotion was prepared using the similar procedure as Example 10.
Details of the respective components used to prepare the milky lotions including the astaxanthin-containing compositions of Examples 17 to 18 are as follows:

- Squalane (trade name, NIKKOL, purified olive squalane, LogP = 12.5, manufactured by NIKKO CHEMICALS CO., LTD.); and
- Cetyl ethylhexanoate (trade name, CEH, LogP = 4.9 or more, manufactured by KOKYU ALCOHOL KOGYO CO., LTD.)

(Stability of astaxanthin-containing composition prepared as emulsion composition)

The storage stability of the obtained milky lotions including the astaxanthin-containing compositions of Examples 17 and 18 was evaluated as shown in Evaluation (4). The evaluation method and evaluation criteria are as described below.

<Evaluation (4): residual-ratio of lycopene>

Immediately after preparation and after storage at 50°C for 7 days, the milky lotions of Examples 10, 17, and 18 were measured by high-speed column chromatography. The measurement result immediately after preparation was defined as 100%, and the residual ratio of lycopene after storage at 50°C for 7 days was calculated. The evaluation results are shown in Table 5.

<Evaluation criteria>

The residual ratio of lycopene was evaluated according to the following evaluation criteria.

Residual ratio of lycopene

s: 80% or more
a: 51 % or more and less than 80%
b: 31% or more and less than 50%
c: less than 31 %

[Table 5]

| | Kinds of oil agent | Evaluation of storage stability (50°C7d) | |
| | | Residual ratio | |
| | | Lycopene | Evaluation |
| --- | --- | --- | --- |
| Example 10 | Bis-ethoxyglycol cyclohexane 1,4-dicarboxylate | 45% | b |
| Example 17 | Squalane | 58% | a |

(continued)

| | Kinds of oil agent | Evaluation of storage stability (50°C7d) | |
|---|---|---|---|
| | | Residual ratio | |
| | | Lycopene | Evaluation |
| Example 18 | Cetyl ethylhexanoate | 60% | a |

The results of Table 5 provided surprising findings such that the residual ratio of lycopene was improved by replacing a part of the oil agents with squalane or cetyl ethylhexanoate.

[Example 19; Milky lotion]

A milky lotion having the following composition was prepared by an ordinary method (the total amount: 100% by mass).

| (Component) | (% by mass) |
|---|---|
| Tomato extract (containing 6% of lycopene) | 0.01 |
| Haematococcus algae extract (containing 20% of astaxanthin) | 0.05 |
| Tocopherol | 1.0 |
| Squalane (LogP = 12.5) | 1.0 |
| Cetyl ethylhexanoate (LogP = 4.9 or more) | 5.0 |
| Meadowfoam seed oil (HLB = 6 or less) | 2.0 |
| Cetyl alcohol (LogP = 5.9) | 1.5 |
| Glycerol monostearate (HLB = 4.8) | 2.0 |
| Polyoxyethylene cetyl ether (HLB = 10.5) | 3.0 |
| Polyoxyethylene sorbitan monooleate (HLB = 10) | 2.0 |
| 1,3-butylene glycol | 1.0 |
| Glycerin | 2.0 |
| Ethylhexyl glycerin (LogP = 1.93) | 0.2 |
| Methylparaben (LogP = 1.96) | 0.3 |
| Sucrose stearate (HLB = 5) | 0.1 |
| Polyglyceryl-10 oleate (HLB = 12) | 0.1 |
| Polyglyceryl-2 stearate (HLB = 5) | 0.1 |
| Phenoxyethanol | 0.2 |
| Collagen | 1.0 |
| Sodium citrate | 1.0 |
| Perfume | suitable amount |
| Purified water | remaining amount |
| Astaxanthin : lycopene : tocopherol = 100 : 6 : 10000 | |

In the formulation, squalane, cetyl ethylhexanoate, meadowfoam oil, cetyl alcohol, glycerol monostearate, polyoxyethylene cetyl ether, polyoxyethylene sorbitan monooleate, ethylhexyl glycerin, methylparaben, sucrose stearate, polyglyceryl-10 oleate, and polyglyceryl-2 stearate correspond to the oil agents in the invention. The amount of the oil agent in the formulation is 17.3% by mass.

[Example 20; cream]

Cream having the following composition was prepared by an ordinary method (the total amount: 100% by mass).

| (Component) | (% by mass) |
|---|---|
| Tomato extract (containing 6% of lycopene) | 0.02 |
| Haematococcus algae extract (containing 20% of astaxanthin) | 0.03 |
| Tocopherol | 2.0 |

(continued)

| (Component) | (% by mass) |
|---|---|
| Cetostearyl alcohol (HLB = 3) | 3.0 |
| Glycerine fatty acid ester (HLB = 9) | 2.0 |
| Polyoxyethylene(20)sorbitan monooleate (HLB = 13) | 1.0 |
| Sorbitan monostearate (HLB = 9) | 1.0 |
| Sodium N-stearoyl-N-methyltaurine | 0.5 |
| Vaseline (LogP = 6.9 or more) | 5.0 |
| Lecithin (HLB = 9) | 0.5 |
| Pentylene glycol | 0.5 |
| Phenoxyethanol | 0.5 |
| 1,3-BG | 1.0 |
| Dimethylpolysiloxane (100 mPa·s) (separated from water) | 3.0 |
| Glyceryl tri-2-ethylhexanoate (HLB = 6.8) | 20.0 |
| Lactic acid | 1.0 |
| Magnesium ascorbyl phosphate | 0.5 |
| Dipropylene glycol | 10.0 |
| Sodium citrate | 0.5 |
| Titanium oxide | 0.1 |
| Perfume | suitable amount |
| Ethyl parahydroxybenzoate (LogP = 1.8) | 0.3 |
| Purified water | remaining amount |
| Astaxanthin : lycopene : tocopherol = 100 : 20 : 33333 | |

In the formulation, cetostearyl alcohol, glycerine fatty acid ester, polyoxyethylene(20)sorbitan monooleate, sorbitan monostearate, vaseline, lecithin, dimethylpolysiloxane (100 mPa·s), glyceryl tri-2-ethylhexanoate, and ethyl parahydroxybenzoate, correspond to the oil agents in the invention. The amount of the oil agent in the formulation is 35.8% by mass.

[Example 21; sunscreen agent]

A sunscreen agent having the following composition was prepared by an ordinary method (the total amount: 100% by mass).

| (Component) | (% by mass) |
|---|---|
| Tomato extract (containing 6% of lycopene) | 0.07 |
| Krill extract (containing 5% of astaxanthin) | 0.05 |
| Tocopherol | 1.0 |
| Squalane (LogP = 12.5) | 3.0 |
| Cyclopentasiloxane (separated from water) | 10.0 |
| Isotridecyl isononanoate (HLB = 2) | 3.0 |
| Titanium oxide | 1.5 |
| Aluminium hydroxide | 1.5 |
| t-butylmethoxydibenzoylmethane (LogP = 4.3) | 4.0 |
| Ethylhexyl methoxycinnamate (LogP = 4.96) | 4.0 |
| PEG-30 dipolyhydroxystearate (HLB = 5) | 0.5 |
| Xanthan gum | 0.05 |
| Mica | 0.2 |
| Iron oxide | 0.01 |
| 1,3-butylene glycol | 1.0 |
| Pentylene glycol | 2.0 |
| Glycerin | 2.0 |

(continued)

| (Component) | (% by mass) |
|---|---|
| Ethylhexyl glycerin (LogP = 1.93) | 0.1 |
| Sucrose stearate (HLB = 5) | 0.1 |
| Lecithin (HLB = 9) | 0.1 |
| Silica | 0.1 |
| Polyglyceryl-10 oleate (HLB = 12) | 0.1 |
| Polyglyceryl-2 stearate (HLB = 5) | 0.1 |
| Phenoxyethanol | 0.2 |
| Hydrolyzed collagen | 1.0 |
| Sodium hyaluronate | 0.5 |
| Perfume | suitable amount |
| Purified water | remaining amount |
| Astaxanthin : lycopene : tocopherol = 100 : 168 : 40000 | |

In the formulation, sualane, cyclopentasiloxane, isotridecyl isononanoate, t-butylmethoxydibenzoylmethane, ethylhexyl methoxycinnamate, PEG-30 dipolyhydroxystearate, ethylhexyl glycerin, sucrose stearate, lecithin, polyglyceryl-10 oleate, and polyglyceryl-2 stearate correspond to the oil agents in the invention. The amount of the oil agent in the formulation is 25% by mass.

[Example 22; moisture facial mask cosmetic]

A moisture facial mask cosmetic having the following composition was prepared by an ordinary method (the total amount: 100% by mass).

| (Component) | (% by mass) |
|---|---|
| Tomato extract (containing 6% of lycopene) | 0.01 |
| Haematococcus algae extract (containing 20% of astaxanthin) | 0.05 |
| Tocopherol | 0.4 |
| Squalane (LogP = 12.5) | 1.0 |
| Isotridecyl isononanoate (HLB = 2) | 2.0 |
| Behenyl alcohol (HLB = 2) | 0.1 |
| Polyoxyethylene cetyl ether (HLB = 10.5) | 1.5 |
| Polyoxyethylene sorbitan monooleate (HLB = 10) | 1.5 |
| 1,3-butylene glycol | 5.0 |
| Glycerin | 4.0 |
| Ethylhexyl glycerin (LogP = 1.93) | 0.2 |
| Methylparaben (LogP = 1.96) | 0.3 |
| Sucrose stearate (HLB = 5) | 0.1 |
| Carbomer | 0.1 |
| Polyglyceryl-2 stearate (HLB = 5) | 0.1 |
| Phenoxyethanol | 0.2 |
| Collagen | 1.0 |
| Sodium citrate | 1.0 |
| Perfume | suitable amount |
| Purified water | remaining amount |
| Astaxanthin : lycopene : tocopherol = 100 : 6 : 4000 | |

In the formulation, squalane, isotridecyl isononanoate, behenyl alcohol, polyoxyethylene cetyl ether, polyoxyethylene sorbitan monooleate, ethylhexyl glycerin, methylparaben, sucrose stearate, and polyglyceryl-2 stearate correspond to the oil agents in the invention. The amount of the oil agent in the formulation is 6.8% by mass.

[Example 23; Jelly-like beauty essence]

A jelly-like beauty essence having the following composition was prepared by an ordinary method (the total amount: 100% by mass).

| (Component) | (% by mass) |
| --- | --- |
| Tomato extract (containing 6% of lycopene) | 0.01 |
| Haematococcus algae extract (containing 20% of astaxanthin) | 0.02 |
| Tocopherol | 0.01 |
| Ceramide III, VI (LogP = 6.6 or more) | 0.01 |
| (PEG-240/decyltetradeceth-20/HDI) Copolymer | 0.5 |
| Hydrolyzed collagen | 1.0 |
| Acetylhydroxyproline | 1.0 |
| Ethylhexyl glycerin (LogP = 1.93) | 0.05 |
| Oleic acid (LogP = 6.36) | 0.05 |
| 1,3-butylene glycol | 1.0 |
| Glycerin | 2.0 |
| Methylparaben (LogP = 1.96) | 0.3 |
| Sucrose stearate (HLB = 5) | 0.01 |
| Polyglyceryl-10 oleate (HLB = 12) | 0.01 |
| Polyglyceryl-2 stearate (HLB = 5) | 0.01 |
| Phenoxyethanol | 0.2 |
| Collagen | 1.0 |
| Sodium citrate | 1.0 |
| Damask rose oil | suitable amount |
| Perfume | suitable amount |
| Purified water | remaining amount |
| Astaxanthin : lycopene : tocopherol = 100 : 15 : 250 | |

In the formulation, ceramide III, ceramide VI, ethylhexyl glycerin, oleic acid, methylparaben, sucrose stearate, polyglyceryl-10 oleate, and polyglyceryl-2 stearate correspond to the oil agents in the invention. The amount of the oil agent in the formulation is 0.44% by mass.

The disclosure of Japanese Patent Application No. 2011-143444 is mentioned in its entirety. All documents, patent applications, and technical standards described in the present specification are incorporated herein by reference to the same extent as if each individual document, patent application, or technical standard were specifically and individually indicated to be incorporated by reference.

Claims

1. An astaxanthin-containing composition comprising: 100 parts by mass of astaxanthin; 1 to 200 parts by mass of lycopene; and 10,000 to 1,000,000 parts by mass of tocopherol where the content ratio of lycopene and tocopherol [(B) : (C)] by mass is from 0.5 : 100000 to 10 : 200.

2. The astaxanthin-containing composition according to claim 1, wherein the lycopene is a fat-soluble extract derived from tomato pulp.

3. The astaxanthin-containing composition according to claim 1 or 2, wherein the astaxanthin-containing composition is an oil composition or an emulsion composition.

4. The astaxanthin-containing composition according to any one of claims 1 to 3, further comprising an oil agent.

5. A cosmetic preparation comprising the astaxanthin-containing composition according to any one of claims 1 to 4.

6. A method for manufacturing an astaxanthin-containing composition comprising:

preparing an oil phase including 100 parts by mass of astaxanthin, 1 to 200 parts by mass of lycopene, and 10,000 to 1,000,000 parts by mass of tocopherol where the content ratio of lycopene and tocopherol [(B) : (C)] by mass is from 0.5 : 100000 to 10 : 200; and mixing the prepared oil phase with an aqueous phase including an aqueous phase component to obtain an emulsion composition.

## Patentansprüche

1. Astaxanthin-enthaltende Zusammensetzung enthaltend: 100 Massenanteile Astaxanthin; 1 bis 200 Massenanteile Lycopen; und 10.000 bis 1.000.000 Massenanteile Tocopherol, wobei das Anteilsverhältnis an Lycopen und Tocopherol [(B):(C)] bezogen auf die Masse von 0,5 : 100000 bis 10 : 200 beträgt.

2. Astaxanthin-enthaltende Zusammensetzung nach Anspruch 1, wobei das Lycopen ein fettlöslicher Extrakt ist, der aus Tomatenmark herrührt.

3. Astaxanthin-enthaltende Zusammensetzung nach Anspruch 1 oder 2, wobei die Astaxanthin-enthaltende Zusammensetzung eine Ölzusammensetzung oder eine Emulsionszusammensetzung ist.

4. Astaxanthin-enthaltende Zusammensetzung nach einem der Ansprüche 1 bis 3, ferner enthaltend ein Ölagens.

5. Kosmetische Zubereitung enthaltend die Astaxanthin-enthaltende Zusammensetzung nach einem der Ansprüche 1 bis 4.

6. Verfahren zur Herstellung einer Astaxanthin-enthaltenden Zusammensetzung umfassend:

   Herstellen einer Ölphase enthaltend 100 Massenanteile Astaxanthin, 1 bis 200 Massenanteile Lycopen und 10.000 bis 1.000.000 Massenanteile Tocopherol, wobei das Anteilsverhältnis an Lycopen und Tocopherol [(B):(C)] bezogen auf die Masse von 0,5:100000 bis 10:200 beträgt; und Mischen der zubereiteten Ölphase mit einer wässrigen Phase, die eine Wässrige-Phase-Komponente enthält, um eine Emulsionszusammensetzung zu erhalten.

## Revendications

1. Composition contenant de l'astaxanthine, comprenant : 100 parties en masse d'astaxanthine ; 1 à 200 parties en masse de lycopène, et de 10 000 à 1 000 000 parties en masse de tocophérol, où le rapport de contenu du lycopène et du tocophérol [(B) : (C)] en masse est compris dans la plage de 0,5 : 100 000 à 10 : 200.

2. Composition contenant de l'astaxanthine selon la revendication 1, dans laquelle le lycopène est un extrait liposoluble dérivé de la pulpe de tomates.

3. Composition contenant de l'astaxanthine selon la revendication 1 ou 2, dans laquelle la composition contenant de l'astaxanthine est une composition d'huile ou une composition d'émulsion.

4. Composition contenant de l'astaxanthine selon l'une quelconque des revendications 1 à 3, comprenant en outre un agent de type huile.

5. Préparation cosmétique comprenant la composition contenant de l'astaxanthine selon l'une quelconque des revendications 1 à 4.

6. Procédé destiné à fabriquer une composition contenant de l'astaxanthine, comprenant :

   la préparation d'une phase huileuse incluant 100 parties en masse d'astaxanthine ; 1 à 200 parties en masse de lycopène, et de 10 000 à 1 000 000 parties en masse de tocophérol, où le rapport de contenu du lycopène et du tocophérol [(B) : (C)] en masse est compris dans la plage de 0,5 : 100 000 à 10 : 200, et le mélange de la phase huileuse préparée avec une phase aqueuse incluant un composant à phase aqueuse pour obtenir une composition d'émulsion.

## REFERENCES CITED IN THE DESCRIPTION

**Patent documents cited in the description**

- JP H249091 A
- JP H5155736 B
- JP 2005047860 A
- JP 2005028559 A
- JP 2007160287 A
- JP 2000229827 A
- JP 5068585 A
- JP 2049091 A
- JP 2011143444 A